# FASCICULE DE BREVET EUROPEEN

(11) **EP 1 983 343 B1**
(45) Date de publication et mention de la délivrance du brevet: **04.05.2011**
(21) Numéro de dépôt: 08003925.8
(22) Date de dépôt: 13.09.2002
(51) Int. Cl.: C12Q 1/04, C12M 1/12, G01N 33/569

(54) **Utilisation d'un dispositif de concentration et de détection de germes pathogènes à partir de produits sanguins et/ou de leurs dérivés**
Verwendung einer Vorrichtung zur Konzentration und Erkennung von pathogenen Keimen aus Blutprodukten und/oder ihren Derivaten
Use of a device of concentrating and detecting pathogenic germs from blood products and/or their derivatives

(30) Priorité: 13.09.2001 FR 0111873
(43) Date de publication de la demande: 22.10.2008
(62) Demande divisionnaire de: 02783159.3
(73) Titulaire: GeneOhm Sciences, Inc., San Diego CA 92121 (US)
(72) Inventeur: Hermet, Jean-Pierre, 92100 Boulogne-Billancourt (FR); Godfrin, Yann, 69009 Lyon (FR); Besson-Faure, Isabelle, 91330 Yerres (FR); Monnot des Angles, Anne, 71100 Saint-Remy (FR); Ribault, Sébastien, 13380 Plan-De-Cuques (FR)
(74) Mandataire: Novagraaf Technologies

(56) Documents cités:
- WO-A-01/09370
- WO-A-89/04372
- DE-A- 19 801 090
- US-A- 3 635 798
- US-A- 5 302 512
- US-A- 5 766 552
- US-A- 5 897 993

## Description

La présente invention a pour objet l'utilisation d'un dispositif pour la mise en oeuvre d'un procédé de concentration de germes pathogènes éventuellement présents dans les produits sanguins ou dérivés, et la détection desdits germes ainsi concentrés pour effectuer un contrôle de la pathogénicité desdits produits sanguins.

On entend par produit sanguin tant le sang total, comme toute préparation issue du fractionnement de celui-ci, comprenant ou non des composants cellulaires, à titre d'exemple, on entend par produit sanguin les concentrés des globules rouges ou de plaquettes, mais aussi les préparations plasmatiques ou sériques.

La détection de contaminations des produits sanguins et dérivés par différents germes pathogènes, tels que des bactéries, des virus, des moisissures, des levures ou autres, est l'une des grandes difficultés auxquelles sont confrontées de nos jours les autorités responsables de la santé publique, ainsi que les industries de la transfusion sanguine.

Des tests de détection existent, mais ils ne sont pas utilisables en routine à ce jour.

US3635798 décrit un dispositif de concentration de micro-organismes présents dans le sang caractérisé par un réservoir contenant un agent d'agglomération des cellules sanguines, un premier filtre dont la taille des pore est comprise entre 8 et 10µm, et un deuxième filtre avec des pores dont la taille est comprise entre 0,40 et 0,50µm capable de retenir les éventuels germes contaminants, le tout connecté par des connexions étanches et stériles.

DE19801090 décrit un dispositif d'isolation/concentration/détection de micro-organismes présent dans le sang comprenant un réservoir contenant un agent de lyse de cellule sanguine et un filtre pour isoler et concentrer les bactéries.

US 5,897,993 décrit un dispositif de détermination du nombre de bactéries comprenant une cuve tubulaire comprenant un orifice d'entrée à une extrémité, l'autre extrémité permettant une succion par une seringue, la cuve comprenant un colorant, un filtre hydrophobe pour la détection des bactéries et un support de filtre.

Les principales difficultés présentées par la plupart des tests de détection de tels germes pathogènes, parmi une population ou une sous-population de cellules sanguines, réside dans le fait que la plupart de traitements censés extraire sélectivement les germes pathogènes, provoquent simultanément une élimination de ceux-ci.

Cette élimination conduit presque systématiquement à une sous-évaluation de la présence desdits germes dans le produit sanguin testé, et donc à une augmentation du risque sanitaire.

La demanderesse a donc conçu une nouvelle méthode rapide et sensible pour détecter une contamination d'un produit sanguin ou dérivé, par de tels germes pathogènes. Pour la mise en oeuvre de cette méthode de détection de germes pathogènes, la présente invention se rapporte à l'utilisation d'un dispositif tel que défini ci-dessous.

L'objet selon de l'invention est remarquable en ce qu'il est réalisé directement sur un échantillon issu d'un produit sanguin prélevé chez un sujet, sans traitement ou dilution préliminaire.

Le procédé de détection de germes pathogènes mis en oeuvre par la Demanderesse consiste essentiellement à concentrer sélectivement lesdits germes pathogènes, puis à les détecter, une fois concentrés, par des techniques connues de l'homme du métier.

La concentration sélective des germes pathogènes est effectuée par élimination séquentielle ou simultanée des différentes populations de cellules sanguines présentes dans un échantillon d'un produit sanguin.

Le procédé de concentration de germes pathogènes mis en oeuvre selon l'invention comporte une première étape de concentration des germes pathogènes consistant à réduire la concentration des populations des cellules sanguines au moyen d'une agrégation sélective de celles-ci, suivie d'une filtration afin de recueillir dans le filtrat les germes pathogènes concentrés non agrégés et de retenir sur le filtre les agrégats de cellules sanguines.

On entend par agrégation, au sens de la présente invention, toute action conduisant à la formation d'agrégats cellulaires, et on entend par agrégat cellulaire tout groupement de cellules comportant plus de deux cellules et dont la taille est supérieure à celle d'une cellule isolée. Au sens de la présente invention, un agrégat peut être obtenu soit par agrégation, telle que l'agrégation des plaquettes subséquente à leur activation, une agglutination, telle que l'agglutination des globules rouges obtenue lors que ceux-ci se trouvent en présence de molécules particulières, soit un rapprochement des cellules induit par un changement de la charge électrostatique de leurs membranes ou encore d'autres mécanismes d'adhésion ou de rapprochement cellulaire conduisant au regroupement de plus de deux cellules.

Selon des modes de mise en oeuvre préférés, l'agrégation des différentes populations de cellules sanguines peut être effectuée à l'aide de composés induisant l'agrégation plaquettaire, ou bien à l'aide de composés induisant l'agglutination spécifique des globules rouges.

On connaît par exemple que, en présence de certains composés, les plaquettes ont la capacité de s'agréger. Ces agrégats peuvent être facilement séparés des germes pathogènes par filtration.

Les globules rouges présentent également quelques propriétés d'agglutination.

Le procédé de concentration de germes pathogènes mis en oeuvre selon l'invention comporte éventuellement une deuxième étape de réduction de la concentration des populations des cellules majoritaires dans le sang, à savoir les plaquettes et les globules rouges, consistant à lyser les cellules isolées non agrégées lors de la première étape d'agrégation.

Cette deuxième étape de réduction de la concentration des populations des cellules sanguines permet une réduction de l'ordre de 4 log (depuis 10⁹ à 10⁵ cellules/ml) en ce qui concerne la concentration des plaquettes et de l'ordre de 5 log (depuis 10¹⁰ à 10⁵) en ce qui concerne la concentration des globules rouges.

Plus précisément, l'invention a pour objet l'utilisation d'un dispositif pour la concentration de germes contaminants éventuellement présents dans un produit sanguin comprenant des cellules sanguines caractérisé en ce qu'il comprend :
- un premier réservoir (1) étanche et stérile contenant au moins un agent d'agrégation de cellules sanguines et éventuellement au moins un agent de marquage de germes pathogènes,
- un deuxième réservoir (2) étanche et stérile contenant au moins un agent de lyse de cellules sanguines et éventuellement au moins un agent de marquage de germes pathogènes,
- un premier filtre (3) placé entre le premier et le deuxième réservoir capable de retenir les agrégats formés dans ledit premier réservoir,
- un deuxième filtre (4) placé en aval du deuxième réservoir capable de retenir les éventuels germes contaminants,
- des moyens de connexion (5) étanches et stériles placés, entre le premier réservoir et le premier filtre, entre le premier filtre et le deuxième réservoir et entre le deuxième réservoir et le deuxième filtre,
pour la mise en oeuvre d'un procédé de concentration de germes contaminants éventuellement présents dans un produit sanguin comprenant des cellules sanguines, comprenant les étapes suivantes :
a) on soumet un échantillon dudit produit sanguin à un traitement d'agrégation des cellules sanguines,
b) on élimine les agrégats formés à l'étape (a) par passage de l'échantillon traité sur un premier filtre laissant passer les germes contaminants mais pas les agrégats de cellules,
c) on lyse sélectivement les cellules résiduelles du filtrat obtenu à l'étape (b),
d) on récupère les germes contaminants par passage du lysat de l'étape (c) sur un second filtre laissant passer des débris cellulaires,
e) on ajoute un agent de marquage des germes contaminants, soit lors de l'agrégation de l'étape (a), soit lors de la lyse de l'étape (c), et
f) on analyse le second filtre pour détecter les germes contaminants marqués éventuellement retenus.

Selon un mode de réalisation préféré, le procédé mis en oeuvre comprend une étape supplémentaire d'analyse du second filtre pour détecter les germes contaminants éventuellement retenus.

Alternativement, le procédé de détection mis en oeuvre comprend l'addition d'un agent de marquage des germes contaminants, directement sur le deuxième filtre lors de l'étape (f) d'analyse.

A titre d'exemple d'agent de marquage des germes pathogènes détectables par le procédé mis en oeuvre selon l'invention, on peut citer une solution de marquage comprenant un substrat estérasique tel que le ChemChrome V6.

Aussi, en tant d'agent de marquage des germes pathogènes détectables par le procédé mis en oeuvre selon l'invention, on peut utiliser une solution de marquage comprenant un anticorps marqué ou un marqueur d'acides nucléiques. Préférentiellement, le marqueur sera fluorescent ou couplé à un fluorochrome ou à un enzyme permettant la dégradation d'un substrat rendu ainsi fluorescent, éventuellement la fluorescence sera détectée par une excitation laser.

Le procédé de détection mis en oeuvre selon l'invention comprend également l'addition d'un agent de perméabilisation des germes contaminants, qui peut être ajouté à au moins l'une des étapes du procédé, soit lors de l'agrégation de l'étape (a), soit lors de la lyse de l'étape (c), soit directement sur le deuxième filtre lors de l'étape (e) d'analyse, soit à plusieurs d'entre elles.

A titre d'exemple d'agent de perméabilisation des germes contaminants, on peut citer le polyéthylènimine, le diacétate de chlorhexidine, le digluconate de chlorhexidine, l'acide d'éthylène diamine tetracétate (EDTA) seul ou en combinaison avec la nisine ainsi que les détergents de type N-Octyl (β-D-glucopyranoside, SDS, Tween, triton, Brij, etc.

Selon un mode particulier de mise en oeuvre du procédé, les cellules sanguines du produit sanguin sont des plaquettes ou des globules rouges ou un mélange de ceux-ci.

Selon un autre mode particulier de mise en oeuvre du procédé, les cellules sanguines du produit sanguin sont des plaquettes et le traitement d'agrégation de l'étape (a) comprend la mise en contact de l'échantillon avec une composition d'agrégation comprenant au moins l'un des agents d'agrégation choisis dans le groupe comprenant :
- un anticorps spécifique d'un antigène plaquettaire,
- un agoniste fort de l'activation plaquettaire, choisi parmi : la thrombine, le TRAP (thrombin receptor activating peptide), la trypsine, le collagène, le thromboxane A2, le PAF (platelet activating factor), le ionophore A23187.
- un agoniste faible de l'activation plaquettaire, choisi parmi ADP, adrénaline, acide arachidonique, facteur Von Willebrand, sérotonine ou épinéphrine.

Avantageusement la concentration en anticorps de type CD9 spécifique d'un antigène plaquettaire dans la composition d'agrégation est comprise entre 0,5 µg/ml et 100 µg/ml, et de préférence elle est comprise entre 5 µg/ml et 40 µg/ml.

Encore avantageusement, la concentration en agoniste fort dans la composition d'agrégation est comprise entre :
- 0,5 UI/ml et 100 UI/ml et de préférence entre 1 UI/ml et 20 UI/ml pour un agoniste de type thrombine,
- 5 µM et 200 µM et de préférence entre 10 et 100 µM pour un agoniste de type TRAP,
- 1 nM et 500 nM et de préférence entre 10 nM et 300 nM pour un agoniste de type trypsine,
- 0,05 µg/ml et 50 µg/ml et de préférence entre 1 µg/ml et 20 µg/ml pour un agoniste de type collagène,
- 0,01 µg/ml et 5 µg/ml et de préférence entre 0,1 et 1 µg/ml pour un agoniste du type thromboxane A2,
- 0,005 mg/ml et 1 mg/ml et de préférence entre 0,05 et 0,5 mg/ml pour un agoniste du type PAF,
- 0,1 µM et 100 µM et de préférence entre 1 µM et 20 µM pour un agoniste du type ionophore A23187.

Avantageusement la concentration en agoniste faible dans la composition d'agrégation est comprise entre :
- 0,5 µM et 100 µM et de préférence entre 1 µM et 20 µM pour un agoniste du type ADP,du type adrénaline ou du type épinéphrine.
- 0,001 mM et 10 mM et de préférence entre 0,01 et 5 pour un agoniste du type acide arachidonique,
- 0,001 mg/ml et 1 mg/ml et de préférence entre 0,01 mg/ml et 0,5 mg/ml pour un agoniste du type facteur Von Willebrand,
- 0,05 µM et 100 µM et de préférence entre 0,01 µM et 50 µM pour un agoniste du type sérotonine.

D'une manière tout à fait préférentielle l'anticorps spécifique d'un antigène plaquettaire, est choisi parmi : un anticorps anti-CD9, CD32, anti-PTA1, CD42, anti-GpIIb/IIIa ou anti-GpIV.

Selon un autre mode particulier de mise en oeuvre du procédé, le produit sanguin comprend des globules rouges et le traitement d'agrégation de l'étape (a) comprend la mise en contact de l'échantillon avec une composition d'agglutination comprenant au moins un agent d'agglutination choisi parmi les lectines, le polyéthylènimine, le polyvinylpyrrolidone (PVP), les gélatines, les dextrans ou les polyéthylènes glycols (PEG).

Avantageusement lesdites lectines présentent une activité érythroagglutinine.

Tout préférentiellement les lectines sont choisies parmi les lectines de *Phaseolus vulgaris, Vicia sativa, Vicia faba* ou *Erythrina corallodendron, Lens culinaris, Phytolacca Americana, Triticum vulgaris.*

Avantageusement la concentration de lectine de type *Phaseolus vulgaris* dans la composition d'agglutination est comprise entre 10 µg/ml et 200 µg/ml.

Avantageusement la concentration de polyéthylènimine dans la composition d'agglutination, est comprise entre 0.1% (poids/volume) et 40% (poids/volume)

Tout préférentiellement lesdits dextrans sont choisis parmi le Dextran 70, Dextran 100, Dextran 500, etc.

Avantageusement la concentration de dextran dans la composition d'agglutination est comprise entre 0.1% (poids/volume) et 40% (poids/volume).

Tout préférentiellement lesdits PEG sont choisis parmi les PEG35, PEG, etc.

Avantageusement la concentration de PEG dans la composition d'agglutination est comprise entre 0.05% (poids/volume) et 40% (poids/volume).

Avantageusement la concentration de gélatine dans la composition d'agglutination est comprise entre 0.5% (poids/volume) et 40% (poids/volume).

Tout préférentiellement lesdites PVP sont choisies parmi les PVP-40, PVP-360, etc.

Avantageusement la concentration de PVP dans la composition d'agglutination est comprise entre 0.05% (poids/volume) et 40% (poids/volume).

Avantageusement, la lyse des cellules de l'étape (c) est réalisée avec une solution de lyse comprenant un ou plusieurs détergents choisis dans le groupe comprenant : saponine, SDS, Tween 20, Triton X100, Brij 96, Polidocanol, N-Octyl β-D-glucopyranoside ou Carbonate de sodium.

De préférence, la solution de lyse est constituée d'un mélange de saponine, de Triton X100 et de Tween 20, et tout préférentiellement la solution de lyse comprend de la saponine à une concentration (exprimée en % poids/volume) comprise entre 0,005% et 0,5%, du Triton X100 à une concentration (exprimée en % poids/volume) comprise entre 0,001% et 0,5% du Tween 20 à une concentration comprise entre 0,01% et 1%, du N-Octyl β-D-glucopyranoside à une concentration comprise entre 0,1% et 0,5%.

Avantageusement, la perméabilisation des bactéries est réalisée avec une solution comprenant un ou plusieurs réactifs choisis dans le groupe comprenant : Chlorhexidine (digluconate, diacétate), Polyéthylènimine, N-Octyl β-D-glucopyranoside, Nisin seule ou en combinaison avec de l'EDTA.

De préférence, les agents perméabilisants sont utilisés pour la chlorhexidine à une concentration (poids/volume) comprise entre 0,0001% (poids/volume) et 0,1% (poids/volume), pour le polyéthylènimine une concentration comprise entre 5 µg/ml et 120 µg/mL, pour leN-Octyl β-D-glucopyranoside à une concentration comprise entre 0,1% (poids/volume) et 0,5% (poids/volume) et pour la Nisin entre 0.1 µg/mL et 0,5 µg/mL seule ou en combinaison avec de l'EDTA à une concentration comprise entre 1 mM et 10 mM.

Le procédé mis en oeuvre selon l'invention peut être utilisé pour concentrer et détecter de nombreux germes contaminants des produits sanguins, parmi lesquels on peut citer des bactéries aérobies ou anaérobies, des moisissures, des levures, des spores de bactéries vivantes et/ou mortes.

Avantageusement la taille des pores du premier filtre mis en oeuvre dans le procédé est comprise entre 2 µm et 20 µm.

Avantageusement la taille des pores du second filtre mis en oeuvre dans le procédé est comprise entre 0,2 µm et 2 µm.

Avantageusement la détection des germes contaminants de l'étape (e) du procédé mis en oeuvre selon l'invention est réalisée dans un dispositif clos.

Tout préférentiellement, les germes contaminants capables d'être concentrés selon le procédé mis en oeuvre selon l'invention, sont choisis parmi le groupe des bactéries aérobies ou anaérobies, des moisissures, des levures ou des spores de bactéries vivantes et/ou mortes, la taille des pores du premier filtre est comprise entre 2 µm et 20 µm, et la taille des pores du second filtre est comprise entre 0,2 µm et 2 µm.

Selon un mode de mise en oeuvre préféré, le dispositif utilisé comprend des moyens de connexion(6) étanches et stériles pour relier la poche contenant le produit sanguin au premier réservoir (1) stérile.

Avantageusement, la connexion (6) étanche et stérile reliant la poche contenant le produit sanguin au premier réservoir stérile est munie d'une valve anti-retour (7).

Selon un autre mode de réalisation préférée, le dispositif utilisé comprend des moyens d'échantillonnage d'un volume déterminé du produit sanguin directement à partir d'une poche de stockage dudit produit vers ledit premier réservoir (1).

Avantageusement, le premier réservoir (1) étanche et stérile du dispositif utilisé est muni d'un système d'aspiration (8) de l'échantillon, et de préférence ledit système d'aspiration est un piston.

Selon un autre mode de mise en oeuvre préféré, le deuxième filtre (4) du dispositif utilisé est inclus dans un support de membrane composé de deux parties pouvant être dissociées permettant de récupérer ledit filtre.

Avantageusement le dispositif utilisé est clos et stérile.

D'autres avantages et caractéristiques de l'invention apparaîtront à la lecture des exemples qui suivent et des figures en annexe dans lesquelles :
- la figure 1 illustre le dénombrement des plaquettes après avoir mis en contact les concentrés plaquettaires avec différentes concentrations de thrombine.
- La figure 2 illustre le dénombrement des plaquettes après agrégation en présence d'ADP pour 2échantillons plaquettaires.
- La figure 3 montre le dénombrement des plaquettes en présence d'anticorps CD9 (clone SN4) pour 4 échantillons plaquettaires.
- La figure 4 montre les résultats du dénombrement des plaquettes en présence de doses

croissantes d'anticorps CD9 (clone 6B1) utilisé sur 3 mL de concentré plaquettaire.
- La figure 5 montre la courbe dose réponse obtenue en présence de concentrations croissantes d'anticorps CD9 (clone SN4).
- La figure 6 illustre l'agglutination de globules rouges en présence de la lectine *Phaseolus vulgaris* utilisée à une concentration de 200 µg/ml pour 3 échantillons de concentrés de globules rouges.
- La figure 7 montre les effets de la solution de lyse sur la récupération de différents germes pathogènes dans des cultures pures.
- La figure 8 montre l'effet de la solution de lyse sur la numération plaquettaire de deux échantillons de plaquettes.
- La figure 9 illustre l'effet de la lyse sur la numération érythrocytaire de deux échantillons différents de concentrés de globules rouges.
- La figure 10 illustre le dénombrement des bactéries Escherichia coli au sein d'un échantillon plaquettaire en utilisant le polyéthylène imine (PEI) en tant qu'agent perméabilisant pour augmenter la pénétration du marqueur.
- La figure 11 illustre l'effet de la concentration en N-octyl β D-glucopyranoside dans la solution de lyse sur des cultures de bactéries pures.

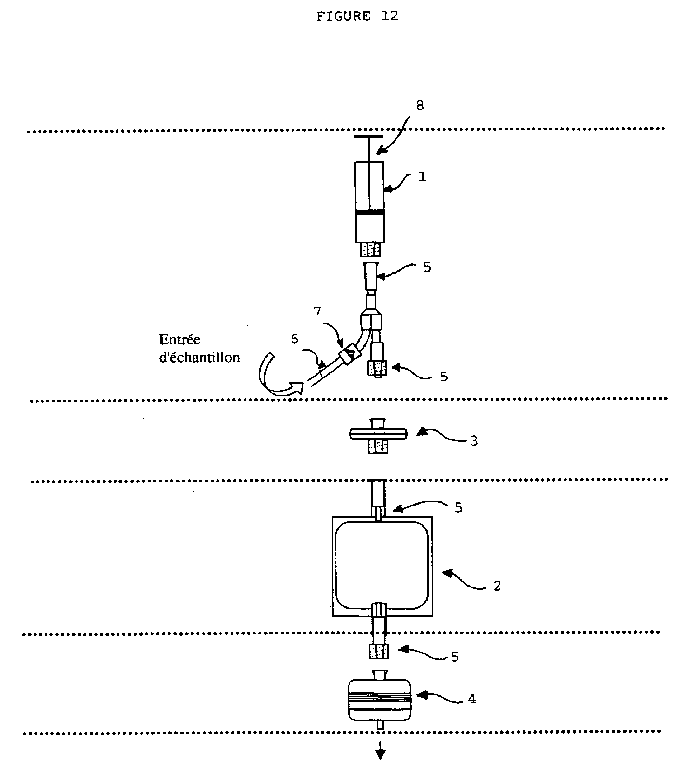

- La figure 12 illustre une mise en oeuvre particulière du dispositif de concentration des germes pathogènes mis en oeuvre selon l'invention, comprenant:
- un premier réservoir (1) étanche et stérile,
- un deuxième réservoir (2) étanche et stérile,
- un premier filtre stérile (3) placé entre le premier réservoir (1) et le deuxième réservoir (2),
- un deuxième filtre stérile (4) placé en aval du deuxième réservoir (2)
- des moyens de connexion (5) étanches et stériles placés, entre le premier réservoir (1) et le premier filtre (3), entre le premier filtre (3) et le deuxième réservoir (2) et entre le deuxième réservoir (2) et le deuxième filtre (4).
- des moyens de connexion (6) étanches et stériles pour relier la poche contenant le produit sanguin au premier réservoir (1).
- une valve anti-retour (7) placée dans la connexion (6) reliant la poche contenant le produit sanguin au premier réservoir 1.
- un système d'aspiration de l'échantillon (8).

### RESULTATS EXPERIMENTAUX

### I. CONCENTRATION DE GERMES PATHOGENES AU MOYEN D'UNE ETAPE D'AGREGATION

### I.1 Agrégation des plaquettes.

### Exemple 1 . Agrégation avec un agoniste fort: la thrombine.

La thrombine est un agoniste fort de l'agrégation plaquettaire.

Dans les travaux expérimentaux menés par la Demanderesse dans le cadre de la présente invention, des solutions de thrombine (référence T8885 Sigma) ont été préparées : à une concentration de 100 UI/ml lorsqu'elle est utilisée à raison de 10 UI/test et sous forme de solution diluée (100 µl de solution mère de thrombine additionnée de 900 µl de tampon PBS), lorsqu'elle est utilisée à raison de 1 UI/test.

L'agrégation des plaquettes par l'intermédiaire de la thrombine comporte les étapes suivantes :
- On place dans un tube 160 µl de concentré plaquettaire auxquels on ajoute 20 µl de tampon PBS et 20 µl de thrombine
- On agite manuellement les tubes durant 5 minutes à température ambiante.
- On ajoute audits tubes 800 µl de tampon PBS.
- On filtre le contenu des tubes sur un filtre de porosité 11 µm.
- On effectue des dilutions en série 1/20 à 1/10 à partir des filtrats.
- On filtre 100 µl de chaque dilution du filtrat sur une membrane CB04.
- On effectue un marquage estérasique.
- On compte les plaquettes éventuellement retenues sur la membrane.

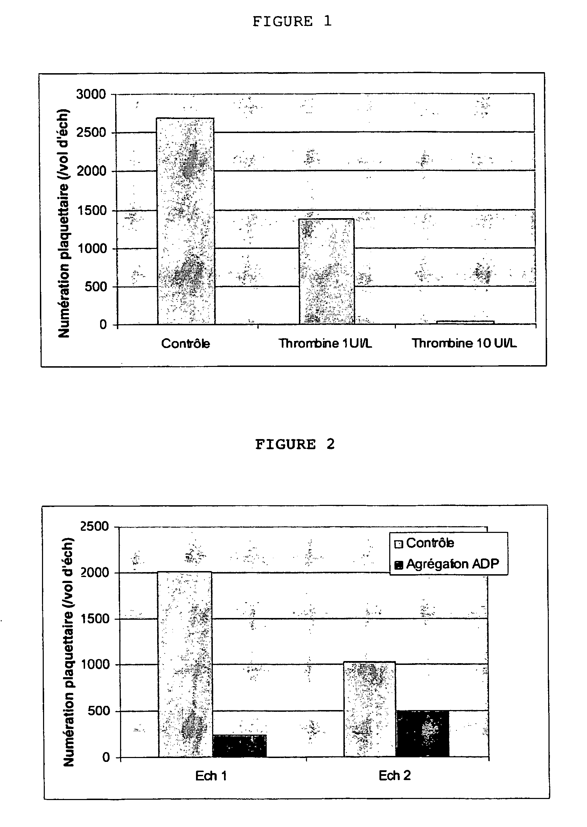

Le tableau 1 ci-dessous illustre les résultats obtenus et représente le nombre d'agrégats plaquettaires obtenus en présence de thrombine utilisée à deux concentrations différentes et la figure 1 en annexe illustre graphiquement ces résultats.

**Tableau 1**

| | Contrôle | Thrombine 1 UI | Thrombine 10 UI |
|---|---|---|---|
| PC 6 | 1584 | 374 | |
| | 1535 | 490 | |
| PC 6 | 2622 | 1358 | 44 |
| | 2737 | 1399 | 30 |

### Exemple 2: Agrégation des plaquettes avec de la thrombine en présence de germes pathogènes.

Les travaux expérimentaux mis en oeuvre afin d'évaluer l'agrégation des germes pathogènes en présence de thrombine comportent les étapes suivantes :
- Une cryosphère de *E. Coli* est introduite dans un tube de 9 ml de bouillon tryptone soja et incubée à 37°C durant 18-24 heures.
- On ajoute à 160 µl de concentré plaquettaire 20 µl de la suspension de *E.coli* et 20µl de thrombine.
- on agite manuellement le tube durant 5 minutes à température ambiante.
- On ajoute 800 µl de tampon PBS,
- On filtre à travers un filtre de porosité 11 µm,
- on effectue des dilutions en série : 1/20 et 1/10 et 1/10,
- On filtre 100 µl de l'échantillon, sur une membrane CB04
- On effectue un marquage estérasique
- On effectue la détection.

Le tableau 2 ci-dessous montre l'agrégation des concentrés plaquettaires avec de la thrombine en présence de *E.coli.*

**Tableau 2**

| Germes pathogènes | Préparation Plaquettes | Thrombine | Résultats Dénombrement |
|---|---|---|---|
| *E. coli* | - | - | 1921 |
| | - | - | 1999 |
| *E. coli* | PC 6 | - | 657 |
| | | | 763 |
| *E. coli* | PC 6 | 1 UI | 140 |
| | | | 167 |
| *E. coli* | PC 6 | 10 UI | 109 |
| | | | 122 |

Après addition de la thrombine, un caillot apparaît presque instantanément.

### Exemple 3: Agrégation des plaquettes en présence d'un agoniste faible: l'ADP.

De l'ADP (SIGMA) est utilisé à une concentration de 200 µM dans de l'eau distillée.

Les travaux expérimentaux mis en oeuvre afin d'évaluer l'agrégation des plaquettes en présence d'ADP comportent les étapes suivantes :
- On introduit dans un tube 400 µl de concentré plaquettaire, auxquels on additionne 50 µl de tampon PBS et 50 µl d'ADP,
- Les tubes sont agités manuellement durant 5 minutes à température ambiante,
- On y ajoute 500 µl de tampons PBS,
- On effectue des dilutions en série de 1/20 et 1/10 et 1/10
- On filtre 100 µl de l'échantillon sur une membrane CB04,
- On effectue un marquage estérasique, et
- On effectue la détection.

Les résultats illustrés sur la figure 1 montrent que, en présence d'ADP, la concentration de plaquettes est réduite d'environ 50% à environ 90%.

### Exemple 4 : Agrégation plaquettaire avec de l'ADP en présence de germes pathogènes.

L'agrégation des plaquettes en présence d'ADP comporte les étapes suivantes :
- Une cryobille de *Staph epidermidis* est introduite dans un tube de 9 ml de bouillon tryptone soja et incubée à 37°C durant 18-24 heures.
- Sur 400 µl de concentré plaquettaire on ajoute 50 µl de la suspension de *Staph epidermidis* et 50 µl d'ADP,
- Les tubes sont agités manuellement durant 5 minutes à température ambiante
- On y ajoute 500 µl de tampon PBS,
- On effectue une filtration sur un filtre de porosité 5 µm,
- On effectue des dilutions en série de 1/20 et 1/10 et 1/10,
- On filtre 100 µl de l'échantillon sur une membrane CB04
- On effectue un marquage estérasique,
- On effectue la détection.

Les germes pathogènes sont ensemencés à une concentration élevée de manière à augmenter un possible effet de piégeage. L'ADP est ajouté aux plaquettes et aux bactéries à une concentration de 10 µM.

**Tableau 3**

| **Germe pathogène** | **Préparation plaquettaire** | **Agrégant** | **Résultats** | **Concentration bactéries/ml** |
|---|---|---|---|---|
| *Staph. epidermidis* | PC 6 | néant | 590 | 6,0E+07 |
| | | | 605 | |
| *Staph. epidermidis* | PC 6 | ADP 10µM | 492 | 4,7E+07 |
| | | | 441 | |

Les résultats obtenus, représentés sur le Tableau 3 ci-dessus montrent que 74% de bactéries sont récupérées après l'étape d'agrégation

### Exemple 5 : Agrégation des plaquettes en présence d'un anticorps CD9.

Il est connu que l'anticorps CD9 induit l'activation des plaquettes et par conséquent leur agrégation. Les tests décrits ont été réalisés avec deux clones CD9, le clone SN4 (Ancel, ref 156-020, concentration 100 µg/ml) et le clone 6B1 (Hemosystem).

Le procédé d'agrégation sélective mis en oeuvre avec cet anticorps CD9 comporte les étapes suivantes :
- Sur 400 µl de concentré plaquettaire on ajoute 50 µl de tampon PBS et 50 µl d'anticorps CD9,
- Les tubes sont agités manuellement durant 5 minutes à température ambiante
- On y ajoute 500 µl de tampon PBS,
- On effectue une filtration sur un filtre de porosité 5 µm
- On effectue des dilutions en série de 1/20 et 1/10 et 1/10,
- On filtre 100 µl de l'échantillon sur une membrane CB04,
- On effectue un marquage estérasique,
- On effectue la détection.

L'anticorps CD9 (clone SN4) a été utilisé en présence des plaquettes à une concentration finale de 10 µg/ml.

La figure 2 en annexe illustre l'agrégation des plaquettes obtenue en présence d'anticorps CD9 (clone SN4).

Afin d'évaluer la concentration optimale de l'anticorps CD9 nécessaire pour l'agrégation plaquettaire, une courbe dose-réponse a été établie.

### Méthode :

- Sur 400 µl de concentré plaquettaire placés dans des tubes on ajoute différentes dilutions d'anticorps, avec des concentrations finales allant de 0 à 20 µg/ml en anticorps CD9.
- Les tubes sont agités manuellement durant 5 minutes à température ambiante
- On y ajoute 500 µl de tampon PBS,
- On effectue une filtration sur un filtre de porosité 5 µm
- On effectue des dilutions en série : 4 dilutions 1/10
- On filtre 100 µl de l'échantillon sur une membrane CB04
- On effectue un marquage estérasique,
- On effectue la détection

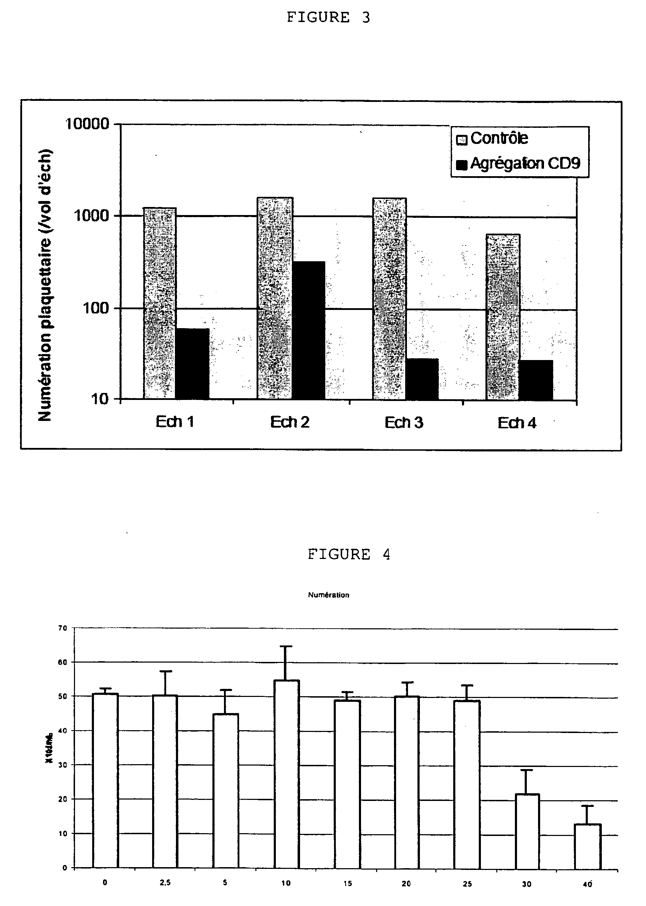

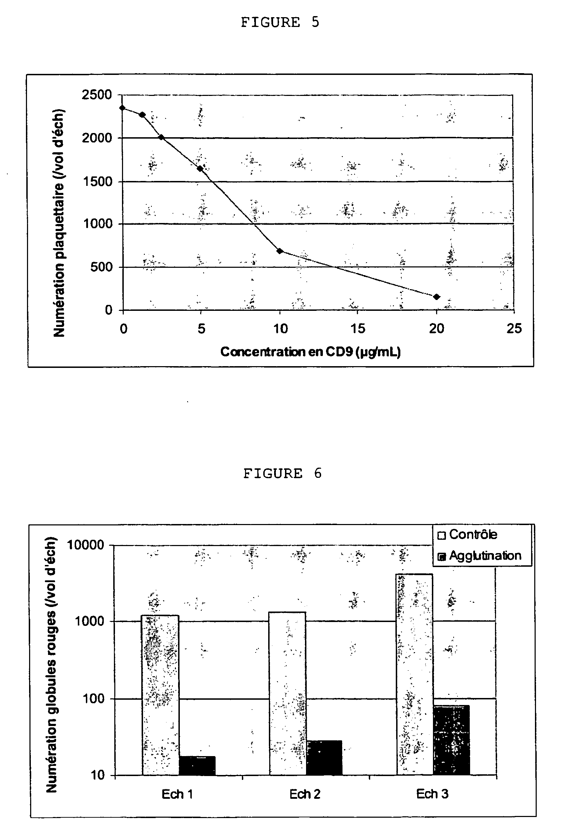

Les figures 3 et 5 montrent respectivement les résultats du dénombrement des plaquettes agrégées en présence de doses croissantes d'anticorps CD9 (clone SN4) et la courbe dose-réponse ainsi obtenue.

Le dénombrement des plaquettes résiduelles est effectué avec l'Analyseur.

L'agrégation est dose-dépendante. Pour augmenter l'effet d'agrégation, la concentration en CD9 a été augmentée autant que possible. Cependant un compromis a été établi pour la détection des bactéries, et l'on a déterminé que la concentration plaquettaire peut être réduite de 1 à 2 Log en utilisant une concentration d'environ 10 µg/ml d'anticorps CD9.

### Exemple 5 bis :

Afin d'évaluer la concentration optimale de l'anticorps CD9 (clone 6B1) nécessaire pour l'agrégation plaquettaire, une expérience du type dose-réponse a été établie

### Méthode :

- Sur 3 mL de concentré plaquettaire placés dans des tubes on ajoute différentes dilutions d'anticorps, avec des concentrations finales allant de 2,5 µg/mL à 40 µg/mL en anticorps CD9. ,
- Les tubes sont agités durant 15 minutes à température ambiante
- On effectue une filtration sur un filtre de porosité 5 µm
- On effectue la numération des plaquettes comme décrit ci-dessus.

La figure 4 en annexe montre les résultats du dénombrement des plaquettes agrégées en présence de doses croissantes d'anticorps CD9 (clone 6B1).

### Exemple 6 : Agrégation plaquettaire avec l'anticorps CD9 en présence de bactéries.

### Méthode :

- Une cryobille de Staph epidermidis est introduite dans un tube de bouillon tryptone soja et incubée à 37°C durant 18-24 heures.
- Sur 400 µl de concentré plaquettaire placés dans des tubes on ajoute 50 µl de la suspension de Staph epidermidis et 50 µl de CD9,
- Les tubes sont agités manuellement durant 5 minutes à température ambiante
- On y ajoute 500 µl de tampon PBS,
- On effectue une filtration sur un filtre de porosité 5 µm
- On effectue des dilutions en série : 4 dilutions 1/10
- On filtre 100 µl de l'échantillon sur une membrane CB04
- On effectue un marquage estérasique.
- On effectue la détection

Le tableau 4 ci-dessous illustre l'agrégation des plaquettes avec du CD9 en présence de bactéries.

**Tableau 4**

| Bactéries | | CD9 | Résultat | % de récupération |
|---|---|---|---|---|
| Staph.epidermidis | PC 10 | - | 46 | |
| | | - | 44 | |
| Staph.epidermidis | PC 10 | + | 43 | 93 |
| | | Agitation | 48 | |
| E. coli | PC 10 | | 218 | |
| | | | 243 | |
| E. coli | PC 10 | CD 9 + | 166 | 64 |
| | | Agitation | 148 | |

Ces résultats montrent que les bactéries Staph epidermidis ont été bien récupérées. Pour E.Coli le dénombrement est réduit de 36%.

### I.2. Agglutination de globules rouges.

Les lectines sont des glycoprotéines d'origine non-immune qui agglutinent les cellules et/ou précipitent des complexes carbohydrates. Ces molécules se lient couramment à des carbohydrates spécifiques.

Dans le cadre de ces travaux expérimentaux, deux lectines ont été utilisées :
- Phaseoulus vulgaris PHA-E
- Vicia Sativa

### Exemple 1 : Agglutination de globules rouges avec des lectines.

La lectine Phaseolus vulgaris PHA-E (Sigma) a été utilisée à une concentration de 2 mg/ml dans du PBS.

La lectine Vicia Activa (Sigma) a été utilisée à la concentration de 1 mg/ml.

Une évaluation rapide des deux lectines a permis de vérifier qu'il n'y a pas d'agglutination des globules rouges avec Vicia sativa. D'autre part Phaseolus vulgaris induit une agglutination rapide et efficace.

### Méthode :

- 400 µl de globules rouges sont placés dans des tubes auxquels on ajoute 50 µl de PBS et 50 µl de Phaseolus vulgaris,
- Les tubes sont agités manuellement durant 5 minutes à température ambiante
- On y ajoute 500 µl de tampon PBS,
- On effectue une filtration sur un filtre de porosité 5 µm,
- On effectue des dilutions en série : 4 dilutions 1/10
- On effectue un marquage avec un anticorps anti-glycophorine-PE.
- On effectue la détection

La figure 6 en annexe montre l'agglutination des globules rouges obtenue en présence de Phaseolus vulgaris.

Dans ce test, Phaseolus vulgaris a été utilisée à une concentration de 200 µg/ml.

On observe une diminution reproductible de deux logs de la concentration en globules rouges en présence de Phaseolus vulgaris.

### Exemple 2 : Agglutination de globules rouges en présence de bactéries.

Les tests préliminaires ont montré qu'il n'y a pas d'interaction entre la lectine Phaseolus et les bactéries.

### Méthode

Une cryobille de E.coli est introduite dans un tube de 9 ml de bouillon tryptone soja et incubée à 37°C durant 18-24 heures.
- On mélange dans des tubes, 400 µl de PBS, 50 µl de E.coli (culture pure) et 50 µl de Phaseolus vulgaris,
- Les tubes sont agités manuellement durant 5 minutes à température ambiante
- On y ajoute 500 µl de tampon PBS,
- On effectue une filtration sur un filtre de 5 µm,
- On effectue des dilutions en série : 4 dilutions 1/10,
- On filtre 100 µl de l'échantillon sur une membrane CB04,
- On effectue un marquage estérasique,
- On effectue la détection.

Les résultats obtenus, en utilisant une concentration Phaseolus vulgaris de 200µg/ml sont illustrés dans le Tableau 5 ci-dessous.

**Tableau 5**

| **Bactéries** | **Globules rouges** | **Lectine (Concentration)** | **Dénombrement** | **% récupération** |
|---|---|---|---|---|
| *E. coli* | RBC 9 | | 662 | |
| NCTC 9001 | | | 604 | |
| *E. coli* | RBC 9 | Phaseolus | 544 | |
| NCTC 9001 | | 200 µg/ml | 579 | 91 |

Ces résultats montrent que 91% des bactéries sont détectées après l'étape d'agglutination et qu'après cette étape d'agglutination, la concentration en globules rouges est réduite de deux logs alors que la souche E. coli est encore bien récupérée.

### II. CONCENTRATION DE GERMES PATHOGENES AU MOYEN D'UNE ETAPE DE LYSE.

Dans le cadre de la présente invention, la demanderesse a effectué une mise au point des différentes techniques de lyse sélective permettant l'élimination des cellules sanguines sans affecter la concentration des bactéries éventuellement présentes dans les échantillons à analyser.

Suite à quelques études préalables, la Demanderesse a constaté que certaines bactéries sont résistantes en présence de détergents tels que le Triton X100 et a déterminé la concentration optimale de détergents avec laquelle le pourcentage de récupération de bactéries est optimal.

### Exemple 1 : Effet de la formulation de la solution de lyse sur des cultures de bactéries pures.

### Méthode

- Les souches sont conservées dans un tube de 9 ml de bouillon tryptone soja et incubées à 37 °C durant 18-24 heures.
- Des dilutions en série (1/10) ont été effectuées dans du tampon PBS jusqu'à 10⁻⁵
- Un millilitre de la dernière dilution est traité avec 9 ml de la solution de lyse (0,01% (poids/volume) de saponine, 0,1% (poids/volume) de Tween et 0,001% (poids/volume) Triton X 100 durant 15 minutes
- 100 µl de l'échantillon sont filtrés sur une membrane CB04
- Un marquage estérasique est effectué selon les indications figurant en annexe 1
- On effectue la détection.

Les résultats de ces différents essais sont illustrés dans le tableau 6 ci-dessous, dans lequel sont exprimés les différents pourcentages de récupération des bactéries obtenus.

**Tableau 6**

| **Souche** | **Contrôle** | **Échantillon lysé** | **% de récupération** |
|---|---|---|---|
| *E. coli* | 132 | 153 | 98 |
| | 145 | 119 | |
| *Bacillus cereus* | 465 | 61 | 11 |
| | 572 | 54 | |
| *E. coli* | 54 | 44 | 83 |
| | 52 | 44 | |
| *Staph. epidermidis* | 2952 | 3034 | 101 |
| | 3069 | 3020 | |
| *E. aerogenes* | 841 | 880 | 110 |
| | 802 | 925 | |
| *Ps. aeruginosa* | 261 | 82 | 31 |
| | 215 | 66 | |
| *Staph. aureus* | 105 | 126 | 125 |
| | 94 | 122 | |
| *P. mirabilis* | 729 | 1129 | 139 |
| | 906 | 1151 | |
| *S. typhymurium* | 608 | 820 | 133 |
| | 600 | 787 | |
| *Serratia marcescens* | 1848 | 1826 | 102 |
| | 1775 | 1860 | |
| *C. amycolatum* | 1103 | 1512 | 156 |
| | 1167 | 2035 | |
| *K. pneumoniae* | 72 | 79 | 99 |
| | 82 | 73 | |
| *P. fluorescens* | 4039 | 4873 | 125 |
| | 3951 | 5105 | |
| *Streptococcus bovis* | 3074 | 1848 | 64 |
| | 2939 | 1978 | |
| *Y. enterocolitica* | 10218 | 9888 | 99 |
| | 10321 | 10526 | |

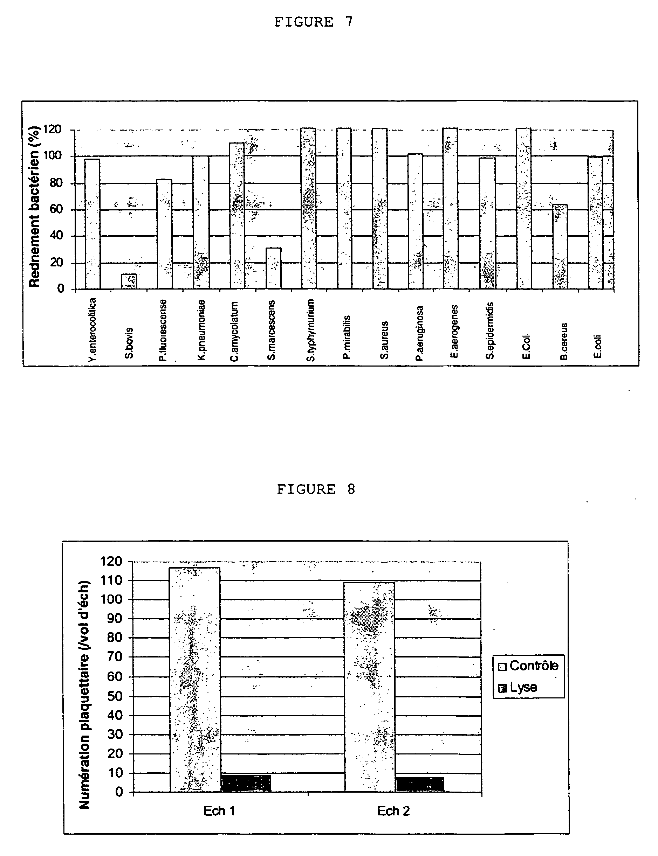

Ces résultats sont illustrés sur la figure 7 en annexe.

La plupart des souches ne sont pas affectées par la solution de lyse: les pourcentages de récupération sont cohérents avec les prévisions prédéfinies, entre 95.et 115%, sauf pour *Ps. aeruginosa* et *Bacillus cereus.*

### Exemple 2 : Effet de la formulation de la solution de lyse sur les bactéries ensemencées dans des plaquettes.

De manière à simuler une contamination, deux souches ont été adaptées à la croissance dans des concentrés plaquettaires.

### Méthode

- Les souches *Bacillus cereus* et *Staph. aureus* ont été ensemencées dans des plaquettes dans des tubes de 50 ml en utilisant une concentration de 10⁵ cellules dans 20 ml de plaquettes.
- Ces tubes de 50 ml sont conservés dans l'incubateur de plaquettes à 22 °C durant plusieurs jours,
- Un millilitre de plaquettes ensemencées est dilué avec 9 ml de la solution de lyse,
- La lyse est effectuée durant 15 minutes à température ambiante,
- 100 µl de l'échantillon sont filtrés sur une membrane CB047,
- Les bactéries sont marquées avec un substrat stéarique comme indiqué en annexe 1,
- La membrane est balayée avec l'Analyseur.

Les résultats du dénombrement des bactéries après la lyse sont illustrés dans le tableau 7 ci-dessous.

**Tableau 7**

| | | **Contrôle** | **Lysés** |
|---|---|---|---|
| Bacillus cereus | | 16 | 14 |
| | | 24 | 27 |
| | bactéries/ml | 2,0E+06 | 2,1E+06 |
| Staph. aureus | | 1966 | 1885 |
| | | 2046 | 1901 |
| | bactéries/ml | 2,0E+08 | 1,9E+08 |

Les bactéries ont été ensemencées à une concentration initiale de 5 10³ cellules/ml et sont détectées plusieurs jours plus tard à des concentrations de l'ordre de 10⁶ à 10⁹ cellules /ml.

Il en résulte de ces expériences, que les bactéries qui se sont développées dans les plaquettes n'ont pas été affectées par la solution de lyse.

### Exemple 2b : Effet de la formulation de la solution de lyse sur des cultures de bactéries pures.

### Méthode

- Les souches sont conservées dans un tube de 9 ml de bouillon tryptone soja et incubées à 37 °C durant 18-24 heures.
- Après détermination du nombre de bactéries par marquage estérasique, 3000 bactéries sont inoculées dans 3 mL de PBS
- Les 3mL sont traités avec la solution de lyse (NOG 0.25% à 2%) pendant 20 minutes
- La totalité de l'échantillon est filtrée sur une membrane CB04
- Le dénombrement est effectué en cytométrie en phase solide

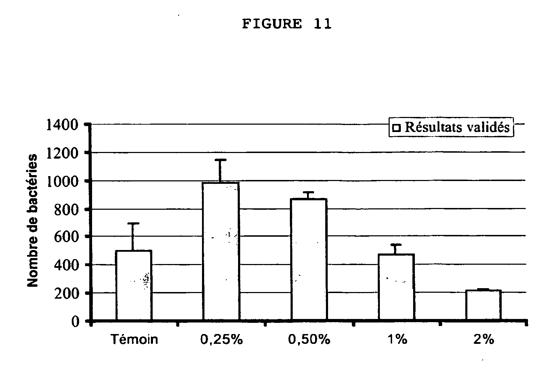

Les résultats obtenus sont illustrés figure 11 en annexe.

### Exemple 3 : Effet de la formulation de la composition de lyse sur les globules rouges.

### Méthode

- Un millilitre de globules rouges est dilué dans 9 ml de solution de lyse ou 9 ml de PBS (contrôle).
- la lyse est effectuée durant 15 minutes à température ambiante
- les échantillons lysés ou non sont analysés à l'aide d'un compteur cellulaire.

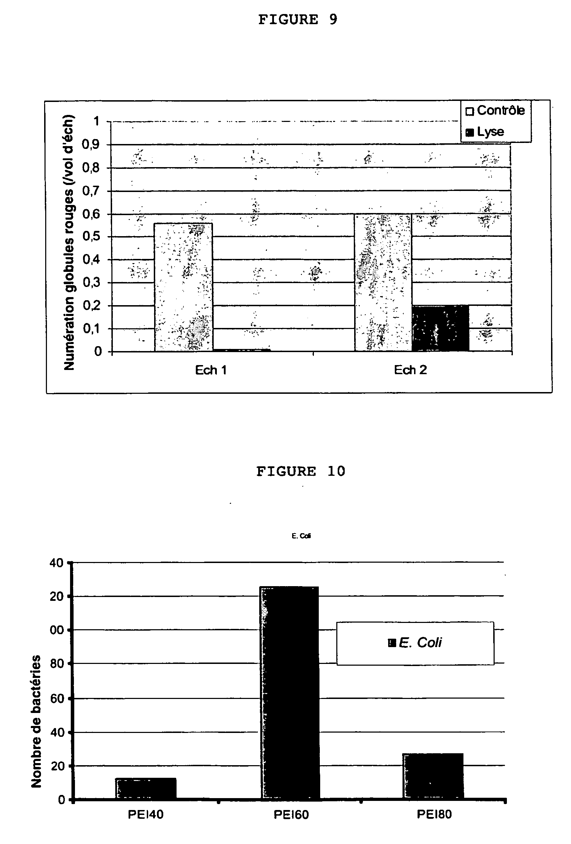

La figure 9 montre les résultats obtenus sur la préparation de globules rouges lysée par rapport à une préparation de globules rouges non lysée.

### Exemple 4 : Effet de la formulation de la composition de lyse sur les plaquettes.

La reproductibilité de l'efficacité de la solution de lyse a été testée. Des échantillons différents de plaquettes ont été lysés et analysés.

### Méthode:

- Un millilitre de plaquettes est dilué dans 9 ml de solution de lyse
- la lyse est effectuée durant 15 minutes à température ambiante
- Des dilutions en série (1/10) ont été effectuées dans du tampon PBS jusqu'à 10⁻⁵
- 100 µl de l'échantillon sont filtrés sur une membrane CB04.
- les microorganismes sont marqués avec un substrat estérasique
- la membrane est balayée avec l'analyseur.

La figure 8 en annexe illustre les résultats de la lyse obtenue avec différents échantillons de plaquettes.

### III. CONCENTRATION DE GERMES PATHOGENES AU MOYEN DE DEUX ETAPES D'AGREGATION ET DE LYSE.

La préparation de l'échantillon par concentration des germes pathogènes en deux étapes comprend :
1) L'Agrégation ou agglutination spécifique des cellules du produit sanguin.
2) La Lyse spécifique des cellules du produit sanguin.

### Exemple 1 hors invention: Séparation spécifique des plaquettes

### Méthode :

- Une cryobille de E.coli est introduite dans un tube de 9 ml de bouillon tryptone soja et incubée à 37°C durant 18-24 heures.

La première étape d'agrégation est effectuée comme suit :
- On mélange dans des tubes, 1 ml de concentré plaquettaire, 50 µl de E.coli (culture pure) et 100 µl de CD 9,
- Les tubes sont agités manuellement durant 5 minutes à température ambiante.

La deuxième étape de lyse est alors effectuée :
- On ajoute 900 µl de tampon de lyse à 100 µl du filtrat après agrégation,
- On maintient l'ensemble 15 minutes à température ambiante,
- On effectue des dilutions en série : 4 dilutions 1/10
- On filtre 100 µl de l'échantillon ainsi obtenu sur une membrane CB04,
- On effectue un marquage des bactéries et des plaquettes avec un substrat estérasique (Annexe 1)
- On dénombre les bactéries et les plaquettes avec l'Analyseur.

Les résultats obtenus en effectuant le procédé de concentration des bactéries en deux étapes sont illustrés dans le tableau 8 ci-dessous.

**Tableau 8**

| **bactéries** | **Concentré plaquettaire (PC)** | **Agent agrégant** | | **Dénombrement bactéries** | **% récupération bactéries** |
|---|---|---|---|---|---|
| *E. coli* | PC | | | 337 | |
| NCTC 9001 | | | | 313 | |
| *E. Coli* | PC | | Lyse | 302 | 86% |
| NCTC 9001 | | | | 256 | |
| *E. Coli* | PC | CD9 | | 266 | 66% |
| NCTC 9001 | | | | 161 | |
| *E. coli* | PC | CD9 | Lyse | 157 | 45% |
| NCTC 9001 | | | | 138 | |

### Exemple 2. Séparation spécifique des globules rouges.

### Méthode :

- Une cryobille de E.coli est introduite dans un tube de 9 ml de bouillon tryptone soja et incubée à 37°C durant 18-24 heures.

L'étape d'agglutination est effectuée comme suit :
- On mélange dans des tubes, 1 ml de globules rouges, 50 µl de E.coli (culture pure) et 125 µl de lectine,
- Les tubes sont agités manuellement durant 5 minutes à température ambiante
- On effectue une filtration sur un filtre de porosité 5 µm.

La deuxième étape de lyse est alors effectuée :
- On ajoute 900 µl de tampon de lyse à 100 µl du filtrat après agglutination,
- On maintient l'ensemble 15 minutes à température ambiante,
- On effectue des dilutions en série : 4 dilutions 1/10
- On filtre 100 µl de l'échantillon ainsi obtenu sur une membrane CB04,
- On effectue un marquage des bactéries avec un substrat estérasique et des globules rouges avec un anticorps anti-Glycophorine-PE (Annexe 2)
- On dénombre les bactéries et les globules rouges avec l'Analyseur.

Le tableau 9 ci-dessous montre les résultats obtenus avec le dénombrement des bactéries effectué après l'étape d'agglutination des globules rouges avec la lectine suivie de l'étape de lyse.

**Tableau 9**

| | | | | **Dénombrement des bactéries** | **récupération des bactéries (%)** | **Dénombrement des globules rouges** |
|---|---|---|---|---|---|---|
| *E. Coli* | Globules rouges 10 | | | 441 | | 3582 |
| NCTC 9001 | | | | 449 | | 4188 |
| *E. coli* | Globules rouges 10 | Lectine | Lyse | 261 | 68 | 161 |
| NCTC 9001 | | | | 348 | | 180 |

Après agglutination la concentration en globules rouges a été réduite de 1,5 log.

Soixante-huit (68) % des bactéries sont récupérées après ces deux étapes.

### IV. AGENTS DE MARQUAGE

### Exemple 1. Substrat estérasique ChemChrome V6

Des solutions de marquage peuvent être préparées à partir d'un substrat estérasique et utilisées dans le procédé de détection mis en oeuvre selon l'invention selon le protocole suivant

### a) Préparation

- 10 µl du substrat estérasique ChemChrome V6 par millilitre de tampon ChemSol B16 (500 µl de solution de marquage par membrane)
- cette solution est conservée à 4°C à l'abri de la lumière au maximum 4 heures.

### b) Utilisation

Introduire un tampon de marquage dans une boîte de Pétri de 33 mm de diamètre.

Distribuer 500 µl de la solution de marquage sur le tampon.

Placer la membrane CB04 sur la rampe de filtration. Filtrer 100 µl de l'échantillon à analyser.

Placer la membrane sur le tampon.

Incuber durant 15 minutes à 37°C.

### Exemple 2. Anticorps marqué.

Aussi, une solution de marquage comprenant un anticorps marqué peut être utilisée dans le procédé de détection mis en **oeuvre** selon l'invention selon le protocole suivant
- Prélever 90 µl d'une dilution de l'échantillon,
- Ajouter 10 µl de l'anticorps anti-glycophorine-PE,
- Vortexer et incuber durant 15 minutes à température ambiante à l'abri de la lumière,
- Ajouter 900 µl de tampon PBS,
- Placer la membrane CB04 dans la rampe de filtration,
- Filtrer sous vide 100 µl de la solution à analyser,
- Analyser la membrane dans l'Analyseur en inversant les câbles primaire et tertiaire de l'Analyseur.

### Exemple 3 : Intérêt de l'ajout d'un agent de perméabilisation des bactéries pour améliorer la pénétration du marqueur.

### Méthode

- Les souches sont conservées dans un tube de 9 ml de bouillon tryptone soja et incubées à 37 °C durant 18-24 heures.
- Après détermination du nombre de bactéries par marquage estérasique, 3000 bactéries sont inoculées dans 3 mL de concentré plaquettaire
- Les 3mL sont traités avec 1 ml de la solution d'agrégation (CD9 :clone 6B1: 30µg/mL, Picogreen 1/2000, PEI 40µg/mL à 80µg/mL) durant 40 minutes
- L'échantillon est filtré au travers d'un filtre de porosité 5 µm
- L'échantillon est incubé dans la solution de lyse (Chlorhexidine 5.10⁻³ %, NOG 0,5%, Nisin 0,2 µg/mL, EDTA 5 mM)) pendant 20 minutes
- La totalité de l'échantillon est filtrée sur une membrane CB04
- Le dénombrement est effectué au moyen d'un analyseur cytomètre en phase solide

### V. CONCLUSIONS

Les options techniques pour atteindre les meilleures conditions de préparation des germes pathogènes ont été définies à l'aide de ses travaux expérimentaux.

En ce qui concerne les concentrés plaquettaires ces options techniques comportent :
1) Etape d'agrégation avec par exemple un anticorps activateur plaquettaire tel que CD9,
2) Etape de lyse cellulaire avec une combinaison des détergents tels que saponine, Tween 20 et Triton X 100.

En ce qui concerne les concentrés de globules rouges :
1) Etape d'agglutination avec une lectine telle que par exemple Phaseolus vulgaris,
2) Etape de lyse cellulaire avec une combinaison de détergents tels que saponine, Tween 20 et Triton X 100.

Le marquage et la perméabilisation des germes pathogènes pouvant intervenir indistinctement lors de l'étape d'agrégation, lors de l'étape de lyse ou directement sur les germes concentrés sur le dernier filtre avant analyse.

### Exemples de modes de réalisation

Point 1) : le procédé de concentration de germes contaminants éventuellement présents dans un produit sanguin comprenant des cellules sanguines mis en oeuvre selon la présente invention dans lequel le procédé comprend les étapes suivantes :
   a) on soumet un échantillon dudit produit sanguin à un traitement d'agrégation des cellules sanguines,
   b) on élimine les agrégats formés à l'étape (a) par passage de l'échantillon traité sur un premier filtre laissant passer les germes contaminants mais pas les agrégats de cellules,
   c) on lyse sélectivement les cellules résiduelles du filtrat obtenu à l'étape (b),
   d) on récupère les germes contaminants par passage du lysat de l'étape (c) sur un second filtre laissant passer les débris cellulaires.
Point 2) : le procédé de détection de germes contaminants dans un produit sanguin comprenant des cellules sanguines, dans lequel on concentre lesdits germes sur un filtre selon les étapes (a) à (d) du procédé du Point 1, puis en ce que :
   (e) on analyse le second filtre pour détecter les germes contaminants éventuellement retenus.
Point 3) : le procédé de détection selon le Point 2 dans lequel on ajoute un agent de marquage des germes contaminants, soit lors de l'agrégation de l'étape (a), soit lors de la lyse de l'étape (c), soit directement sur le deuxième filtre lors de l'étape (e) d'analyse.
Point 4) : Le procédé selon les points 2 ou 3 dans lequel le procédé comprend l'addition d'un agent de perméabilisation des germes contaminants, à au moins l'une des étapes (a), (c), ou (e).
Point 5) : Procédé selon le point 4, dans lequel l'agent de perméabilisation est choisi parmi le groupe comprenant : le polyéthylènimine, le diacétate de chlorhexidine, le digluconate de chlorhexidine, l'acide d'éthylène diamine tetracétate (EDTA) seul ou en combinaison avec la nisine, un détergent ou un mélange de ceux-ci.
Point 6) : le procédé selon le point 5, dans lequel le détergent est choisi parmi le groupe comprenant : N-Octyl β-D-glucopyranoside, SDS, Tween, triton, Brij, ou un mélange de ceux-ci.
Point 7) : le procédé selon l'un quelconque des points 1 à 6, dans lequel l'agent de marquage des germes pathogènes est une solution de marquage choisie parmi le groupe comprenant un substrat estérasique tel que le ChemChrome V6, un anticorps marqué ou un marqueur d'acides nucléiques.
Point 8) : le Procédé selon l'un quelconque des points 3 à 7, dans lequel l'agent de marquage comprend un marqueur fluorescent ou un agent couplé à un fluorochrome ou à un enzyme permettant la dégradation d'un substrat rendu ainsi fluorescent.
Point 9) : le procédé selon le point 8, dans lequel la fluorescence est détectée au moyen d'une excitation laser. Point 10) : le procédé selon l'un des points 1 à 9, dans lequel les cellules sanguines du produit sanguin sont des plaquettes ou des globules rouges ou un mélange de ceux-ci.
Point 11) : le procédé selon l'un des points 1 à 10 dans lequel le produit sanguin comprend des plaquettes et en ce que le traitement d'agrégation de l'étape (a) comprend la mise en contact de l'échantillon avec une composition d'agrégation comprenant au moins l'un des agents d'agrégation choisis dans le groupe comprenant :
   - un anticorps spécifique d'un antigène plaquettaire,
   - un agoniste fort de l'activation plaquettaire choisie parmi : la thrombine, le TRAP (thrombin receptor activating peptide), la trypsine, le collagène, le thromboxane A2, le PAF (platelet activating factor), le ionophore A23187, les complexes immuns et facteurs du complément,
   - un agoniste faible de l'activation plaquettaire choisie parmi ADP, adrénaline, acide arachidonique, facteur Von Willebrand, sérotonine ou épinéphrine.
Point 12) : le procédé selon le point 11, dans lequel la concentration en anticorps de type CD9 dans la composition d'agrégation est comprise entre 0,5 µg/ml et 50 µg/ml, et de préférence entre 5 µg/ml et 40 µg/ml.
Point 13) : le procédé selon le point 11, dans lequel la concentration en agoniste fort dans la composition d'agrégation est comprise entre :
   - 0,5 UI/ml et 100 UI/ml et de préférence entre 1 UI/ml et 20 UI/ml pour un agoniste de type thrombine,
   - 5 µM et 200 µM et de préférence entre 10 µM et 100 µM pour un agoniste de type TRAP,
   - 1 nM et 500 nM et de préférence entre 10 nM et 300 nM pour un agoniste de type trypsine,
   - 0,05 µg/ml et 50 µg/ml et de préférence entre 1 µg/ml et 20 µg/ml pour un agoniste de type collagène,
   - 0,01 µg/ml et 5 µg/ml et de préférence entre 0,1 µg/ml et 1 µg/ml pour un agoniste du type thromboxane A2,
   - 0,005 mg/ml et 1 mg/ml et de préférence entre 0,05 mg/ml et 0,5 mg/ml pour un agoniste du type PAF,
   - 0,1 µM et 100 µM et de préférence entre 1 µM et 20 µM pour un agoniste du type ionophore A23187.
Point 14) : le procédé selon le point 11, dans lequel la concentration en agoniste faible dans la composition d'agrégation est comprise entre :
   - 0,5 µM et 100 µM et de préférence entre 1 µM et 20 µM pour un agoniste du type ADP, du type adrénaline ou du type épinéphrine.
   - 0,001 mM et 10 mM et de préférence entre 0,01 mM et 5 mM pour un agoniste du type acide arachidonique,
   - 0,001 mg/ml et 1 mg/ml et de préférence entre 0,01 mg/ml et 0,5 mg/ml pour un agoniste du type facteur Von Willebrand, ou
   - 0,05 µM et 100 µM et de préférence entre 0,01 µM et 50 µM pour un agoniste du type sérotonine.
Point 15) : le procédé selon le point 11, dans lequel l'anticorps est choisi parmi: un anticorps anti-CD9, anti-CD32, anti-PTA1, anti-D42, anti-GpIIb/IIIa ou anti-GpIV.
Point 16) : le procédé selon l'un quelconque des points 1 à 15, dans lequel le produit sanguin comprend des globules rouges et en ce que le traitement d'agrégation de l'étape (a) comprend la mise en contact de l'échantillon avec une composition d'agglutination comprenant au moins un agent d'agglutination choisi parmi les lectines, le polyéthylènimine, la polyvinylpyrrolidone (PVP), les gélatines, les dextrans ou les polyéthylène glycols (PEG).
Point 17) : le procédé selon le point 16, dans lequel les lectines présentent une activité érythroagglutinine.
Point 18) : le procédé selon les points 16 ou 17, dans lequel les lectines sont choisies parmi les lectines de *Phaseolus vulgaris, Vicia sativa, Vicia faba* ou *Erythrina corallodendron, Lens culinaris, Phytolacca Americana, Triticum vulgaris.*
Point 19) : le procédé selon le point 18, dans lequel la concentration de lectine de *Phaseolus vulgaris* dans la composition d'agglutination est comprise entre 10 µg/ml et 800 µg/ml.
Point 20) : le procédé selon le point 16, dans lequel la concentration de polyéthylènimine dans la composition d'agglutination est comprise entre 0,1% (poids/volume) et 40% (poids/volume) de préférence entre 20% (poids/volume) et 40% (poids/volume).
Point 21) : le procédé selon le point 16, dans lequel la Polyvinylpyrrrolidone (PVP) est choisie parmi le groupe comprenant du PVP-40 et du PVP-360 et utilisée de préférence à une concentration comprise entre 0,1% (poids/volume) et 40% (poids/volume), et tout préférentiellement entre 4% (poids/volume) et 20% (poids/volume).
Point 22) : le procédé selon le point 16 dans lequel la gélatine dans la composition d'agglutination est utilisée de préférence à une concentration comprise entre 0,5% (poids/volume) et 40% (poids/volume), et tout préférentiellement entre 40% (poids/volume) et 20% (poids/volume).
Point 23) : le procédé selon le point 16, dans lequel le dextran dans la composition d'agglutination est choisi parmi le groupe comprenant : Dextran 70, Dextran 100, et Dextran 500, utilisé de préférence à une concentration comprise entre 0,1% (poids/volume) et 40% (poids/volume), et tout préférentiellement entre 4% (poids/volume) et 20% (poids/volume).
Point 24) : le procédé selon le point 16, dans lequel le polyéthylène glycol est choisi parmi le groupe comprenant : PEG8, PEG17, PEG35, utilisé de préférence à une concentration comprise entre 0,05% (poids/volume) et 40% (poids/volume) et tout préférentiellement entre 20% (poids/volume) et 40% (poids/volume).
Point 25) : le procédé selon l'un quelconque des points précédents, dans lequel la lyse des cellules de l'étape (c) est réalisée avec une solution de lyse comprenant un ou plusieurs détergents choisis dans le groupe comprenant : saponine, SDS, Tween 20, Triton X100, Brij 96, Polidocanol, N-octyl β-D-glucopyranoside ou Carbonate de sodium.
Point 26) : le procédé selon l'une quelconque des points précédents, dans lequel les germes contaminants sont des bactéries aérobies ou anaérobies, des moisissures, des levures, des spores de bactéries vivantes et/ou mortes.
Point 27) : le procédé selon l'une quelconque des points précédents, dans lequel la taille des pores du premier filtre est comprise entre 2 µm et 20 µm.
Point 28) : le procédé selon l'un quelconque des points précédents, dans lequel la taille des pores du second filtre est comprise entre 0,2 µm et 2 µm.
Point 29) : le procédé selon l'un quelconque des points précédents, dans lequel les germes contaminants sont des bactéries aérobies ou anaérobies, des moisissures, des levures ou des spores de bactéries vivantes et/ou mortes, en ce que la taille des pores du premier filtre est comprise entre 2 µm et 20 µm, et en ce que la taille des pores du second filtre est comprise entre 0,2 µm et 2 µm.
Point 30) : le procédé selon l'un quelconque des points 2 à 29, dans lequel la détection des germes contaminants de l'étape (e) est réalisée dans un dispositif clos.
Point 31) : le dispositif pour la concentration de germes contaminants éventuellement présents dans un produit sanguin comprenant des cellules sanguines, susceptible d'être mis en oeuvre dans un procédé selon l'une quelconque des points 1 à 30, dans lequel ledit dispositif comprend :
   - un premier réservoir (1) étanche et stérile contenant au moins un agent d'agrégation de cellules sanguines et éventuellement au moins un agent de marquage de germes pathogènes,
   - un deuxième réservoir (2) étanche et stérile contenant au moins un agent de lyse de cellules sanguines et éventuellement au moins un agent de marquage de germes pathogènes,
   - un premier filtre (3) placé entre le premier et le deuxième réservoir capable de retenir les agrégats formés dans ledit premier réservoir,
   - un deuxième filtre (4) placé en aval du deuxième réservoir capable de retenir les éventuels germes contaminants,
   - des moyens de connexion (5) étanches et stériles placés, entre le premier réservoir et le premier filtre, entre le premier filtre et le deuxième réservoir et entre le deuxième réservoir et le deuxième filtre.
Point 32) : le dispositif selon le point 31 dans lequel une connexion (6) étanche et stérile pour relier la poche contenant le produit sanguin au premier réservoir stérile.
Point 33) : le dispositif selon le point 32, dans lequel ladite connexion étanche et stérile reliant la poche contenant le produit sanguin au premier réservoir stérile est munie d'une valve (7) anti-retour.
Point 34) : le dispositif selon les points 31 ou 33 dans lequel ledit dispositif comprend des moyens d'échantillonnage d'un volume déterminé du produit sanguin directement à partir d'une poche de stockage dudit produit vers ledit premier réservoir.
Point 35) : le dispositif selon le point 34, dans lequel le premier réservoir stérile est muni d'un système d'aspiration de l'échantillon (8).
Point 36) : le dispositif selon le Point 35, dans lequel le système d'aspiration est un piston.
Point 37) : le dispositif selon l'un quelconque des points 31 à 36, dans lequel le deuxième filtre est inclus dans un support de membrane composé de 2 parties pouvant être dissociées permettant de récupérer le filtre.
Point 38) : le dispositif selon l'un quelconque des points 31 à 37, dans lequel ledit dispositif est clos et stérile.

## Revendications

1. Utilisation d'un dispositif pour la concentration de germes contaminants éventuellement présents dans un produit sanguin comprenant des cellules sanguines **caractérisé en ce qu'**il comprend :
- un premier réservoir (1) étanche et stérile contenant au moins un agent d'agrégation de cellules sanguines et éventuellement au moins un agent de marquage de germes pathogènes,
- un deuxième réservoir (2) étanche et stérile contenant au moins un agent de lyse de cellules sanguines et éventuellement au moins un agent de marquage de germes pathogènes,
- un premier filtre (3) placé entre le premier et le deuxième réservoir capable de retenir les agrégats formés dans ledit premier réservoir,
- un deuxième filtre (4) placé en aval du deuxième réservoir capable de retenir les éventuels germes contaminants,
- des moyens de connexion (5) étanches et stériles placés, entre le premier réservoir et le premier filtre, entre le premier filtre et le deuxième réservoir et entre le deuxième réservoir et le deuxième filtre,
pour la mise en oeuvre d'un procédé de détection de germes contaminants éventuellement présents dans un produit sanguin comprenant des cellules sanguines comprenant les étapes suivantes :
a) on soumet un échantillon dudit produit sanguin à un traitement d'agrégation des cellules sanguines,
b) on élimine les agrégats formés à l'étape (a) par passage de l'échantillon traité sur un premier filtre laissant passer les germes contaminants mais pas les agrégats de cellules,
c) on lyse sélectivement les cellules résiduelles du filtrat obtenu à l'étape (b),
d) on récupère les germes contaminants par passage du lysat de l'étape (c) sur un second filtre laissant passer les débris cellulaires,
e) on ajoute un agent de marquage des germes contaminants, soit lors de l'agrégation de l'étape (a), soit lors de la lyse de l'étape (c), et
f) on analyse le second filtre pour détecter les germes contaminants marqués éventuellement retenus.

2. Utilisation selon la revendication 1 dans laquelle le dispositif comprend une connexion (6) étanche et stérile pour relier la poche contenant le produit sanguin au premier réservoir stérile.

3. Utilisation selon la revendication 2, dans laquelle ladite connexion étanche et stérile reliant la poche contenant le produit sanguin au premier réservoir stérile est munie d'une valve (7) anti-retour.

4. Utilisation selon les revendications 1 ou 3 dans laquelle le dispostif comprend des moyens d'échantillonnage d'un volume déterminé du produit sanguin directement à partir d'une poche de stockage dudit produit vers ledit premier réservoir.

5. Utilisation selon les revendications 2 ou 4, dans laquelle le premier réservoir stérile est muni d'un système d'aspiration de l'échantillon (8).

6. Utilisation selon la revendication 5, dans laquelle le système d'aspiration est un piston.

7. Utilisation selon l'une quelconque des revendications 1 à 6, dans laquelle le deuxième filtre est inclus dans un support de membrane composé de 2 parties pouvant être dissociées permettant de récupérer le filtre.

8. Utilisation selon l'une quelconque des revendications 1 à 7, dans laquelle le dispositif est clos et stérile.

## Claims

1. Use of a device, for concentrating contaminating germs possibly present in a blood product containing blood cells, **characterized in that** it comprises:
- a first watertight and sterile tank (1) containing at least one blood cell aggregation agent and, optionally at least one contaminating germ-labelling agent,
- a second watertight and sterile tank (2) containing at least one blood cells lysing agent and optionally at least one contaminating germ-labelling agent,
- a first filter (3) localized between the first and second tanks capable of retaining aggregates formed in said first tank,
- a second filter (4) located downstream of the second tank capable of retaining any contaminating germs,
- watertight and sterile connection means (5) placed between the first tank and the first filter, between the first filter and second tank, and between the second tank and the second filter,
for implementing of a method for detecting contaminating germs potentially present in a blood product containing blood cells, **characterised in that** it includes the following steps:
a) subjecting a sample of said blood product to a blood cell aggregation treatment,
b) eliminating the aggregates formed at step (a) by passing the treated sample over a first filter allowing the contaminating germs to pass through but not the cell aggregates,
c) selectively lysing the residual cells of the filtrate obtained at step (b),
d) recovering the contaminating germs by passing the lysate of step (c) over a second filter allowing the cell debris to pass through,
e) adding a contamination germ-labelling agent either during the aggregation step (a), or during the lysis of step (c), and
f) Examining the second filter in order to detect the labelled contaminating germ possibly retained.

2. Use according to claim 1 wherein the device comprises a watertight and sterile connection (6) connecting the bag containing the blood product to the first sterile tank.

3. Use according to claim 2, wherein a said watertight and sterile connection connecting the bag containing the blood product to the first sterile tank is equipped with a anti-return valve (7).

4. Use according to claim 1 or 3 wherein the device comprises sampling means for a determined volume of blood product directly from a storage bag of said product to said first tank.

5. Use according to claims 2 or 4, wherein the first sterile tank is provided with a suctioning system of the sample (8).

6. Use according to claim 5, wherein the suctioning system is a piston.

7. Use according to any of claims 1 to 6, wherein the second filter is included in a support membrane having 2 parts that can be separated for recovering the filter.

8. Use according to any of claims 1 to 7, wherein the device is enclosed and sterile.

## Patentansprüche

1. Verwendung einer Vorrichtung zur Konzentration eventuell in einem Blutprodukt vorhandener kontaminierender Keime, Blutzellen umfassend, **dadurch gekennzeichnet, dass** sie folgendes umfasst:
- einen ersten dichten und sterilen Behälter (1), der mindestens ein Aggregationsmittel von Blutzellen und eventuell mindestens einen Marker für pathogene Keime enthält,
- einen zweiten dichten und sterilen Behälter (2), der mindestens ein Lysemittel von Blutzellen und eventuell mindestens einen Marker für pathogene Keime enthält,
- einen ersten zwischen dem ersten und zweiten Behälter angebrachten Filter (3), der die in dem besagten ersten Behälter gebildeten Aggregate zurückhalten kann,
- einen zweiten nach dem zweiten Behälter angebrachten Filter (4), der gegebenenfalls kontaminierende Keime zurückhalten kann,
- dichte und sterile, zwischen dem ersten Behälter und dem ersten Filter, zwischen dem ersten Filter und dem zweiten Behälter und zwischen dem zweiten Behälter und dem zweiten Filter angebrachte Verbindungsmittel(5) zur Durchführung eines Verfahrens zum Nachweis von eventuell in einem Blutprodukt vorhandenen kontaminierenden Keimen, Blutzellen umfassend, das folgende Schritte umfasst:
a) man unterzieht eine Probe des besagten Blutprodukts einer Aggregationsbehandlung der Blutzellen,
b) man entfernt die in Schritt (a) gebildeten Aggregate durch Passage der behandelten Probe über einen ersten Filter, der die kontaminierenden Keime, aber nicht die Zellaggregate passieren lässt,
c) man lysiert selektiv die residuellen Zellen des in Schritt (b) gewonnenen Filtrats,
d) man gewinnt die kontaminierenden Keime durch Passage des Lysats von Schritt (c) über einen zweiten Filter zurück, der den Zellschrott passieren lässt,
e) man fügt einen Marker für die kontaminierenden Keime hinzu, entweder bei der Aggregation von Schritt (a) oder bei der Lyse von Schritt (c), und
f) man analysiert den zweiten Filter, um die eventuell zurückgehaltenen markierten kontaminierenden Keime nachzuweisen.

2. Verwendung nach Anspruch 1, **dadurch gekennzeichnet, dass** die Vorrichtung eine dichte und sterile Verbindung (6) umfasst, um den das Blutprodukt enthaltenden Beutel mit dem ersten sterilen Behälter zu verbinden.

3. Verwendung nach Anspruch 2, **dadurch gekennzeichnet, dass** besagte dichte und sterile Verbindung, die den das Blutprodukt enthaltenden Beutel mit dem ersten sterilen Behälter verbindet, mit einem Rückschlagventil (7) versehen ist.

4. Verwendung nach Anspruch 1 oder 3, **dadurch gekennzeichnet, dass** die Vorrichtung Mittel zur Stichprobenentnahme eines bestimmten Volumens des Blutprodukts direkt aus einem Speicherbeutel des besagten Produkts zu dem besagten ersten Behälter enthält.

5. Verwendung nach Anspruch 2 oder 4, **dadurch gekennzeichnet, dass** der erste sterile Behälter mit einem Ansaugsystem der Probe (8) versehen ist.

6. Verwendung nach Anspruch 5, **dadurch gekennzeichnet, dass** das Ansaugsystem ein Kolben ist.

7. Verwendung nach einem beliebigen der Ansprüche 1 bis 6, **dadurch gekennzeichnet, dass** der zweite Filter in der Membranhalterung enthalten ist, die aus zwei Teilen besteht, die getrennt werden können, wodurch der Filter entnommen werden kann.

8. Verwendung nach einem beliebigen der Ansprüche 1 bis 7, **dadurch gekennzeichnet, dass** die Vorrichtung geschlossen und steril ist.
